# FASCICULE DE BREVET EUROPEEN

(11) **EP 3 324 901 B1**
(45) Date de publication et mention de la délivrance du brevet: **05.06.2019**
(21) Numéro de dépôt: 16750977.7
(22) Date de dépôt: 19.07.2016
(51) Int. Cl.: A61F 5/455

(54) **COUPE MENSTRUELLE ERGONOMIQUE**
ERGONOMISCHE MENSTRUATIONSSCHALE
ERGONOMIC MENTRUAL CUP

(30) Priorité: 24.07.2015 FR 1557046
(43) Date de publication de la demande: 30.05.2018
(73) Titulaire: Teolab, 92110 Clichy (FR)
(72) Inventeur: MEDAS, Bertrand, 69005 Lyon (FR)
(74) Mandataire: Cabinet Beau de Loménie
(86) Numéro de dépôt international: PCT/FR2016/051845
(87) Numéro de publication internationale: WO 2017/017340

(56) Documents cités:
- WO-A1-2006/058409
- DE-U1-202009 008 893
- US-A- 2 616 426
- US-A- 4 381 771
- US-A- 5 295 984
- US-A1- 2014 012 216
- US-B2- 8 795 248

## Description

### DOMAINE DE L'INVENTION

L'invention concerne une coupe menstruelle. Une telle coupe a vocation à être introduite dans le vagin et être portée par une femme pendant la durée de ses menstruations afin de recueillir le flux menstruel.

Dans ce but, la coupe présente une forme de petite coupe ou coupelle aux bords arrondis, formée en matériau élastique.

Son ouverture en partie haute est délimitée habituellement par un anneau d'étanchéité, qui assure l'étanchéité de la coupe lorsque celle-ci est en place dans le vagin. En dessous de l'anneau, la coupe est en général constituée principalement par une partie inférieure évasée en forme de dôme inversé, qui est reliée à l'anneau d'étanchéité soit directement soit par l'intermédiaire d'une partie de jonction sensiblement cylindrique (appelée ci-après 'partie cylindrique'). La partie inférieure évasée se termine le plus souvent en bas par une petite tige servant à faciliter le retrait de la coupe.

### ARRIERE-PLAN TECHNOLOGIQUE

Le marché de l'hygiène féminine a beaucoup évolué ces dernières années. Par rapport aux tampons hygiéniques ou aux serviettes périodiques, la coupe menstruelle apparaît comme une solution de plus en plus adaptée aux attentes des femmes modernes. Une telle coupe a été divulguée par exemple par le document US 2,616,426.

Cependant, les formes de coupe actuelles laissent encore à désirer quant à la facilité d'insertion et de retrait de la coupe dans le vagin.

L'insertion d'une coupe menstruelle se fait généralement en repliant tout d'abord la coupe sur elle-même. La coupe est alors mise en place dans le vagin, dans lequel elle se déplie et reprend sa forme initiale.

L'élasticité du matériau de la coupe fait que la surface externe de son anneau d'étanchéité se trouve alors plaquée sur la paroi vaginale, ce qui permet que l'anneau est alors relativement étanche. Le flux menstruel peut alors être recueilli dans la cavité intérieure de la coupe.

En général, une coupe menstruelle se termine en bas par une petite tige. Le retrait de la coupe se fait tout d'abord en attrapant celle-ci par la tige. En tirant sur cette tige, on peut dans un premier temps faire descendre la coupe vers l'ouverture du vagin, puis éventuellement retirer la coupe hors du vagin.

Cependant, le fait de tirer sur la tige pour retirer la coupe crée un effet de succion (ou effet « ventouse ») très désagréable, les bords de la coupe venant épouser la paroi vaginale.

L'objectif de la présente invention est de remédier à cette difficulté et de proposer une coupe menstruelle présentant une partie inférieure évasée, de coût de revient modéré, et dont le retrait ne provoque pas de sensations désagréables.

Cet objectif est atteint grâce au fait que la coupe menstruelle comporte une pluralité de surfaces de préhension non convexes, réparties de manière sensiblement axisymétrique au moins sur ladite partie inférieure évasée, et configurées pour pouvoir être pincées entre des doigts afin de faciliter le retrait de la coupe.

Pour des raisons d'ergonomie, de préférence la coupe menstruelle selon l'invention comporte exactement soit deux surfaces de préhension, soit trois surfaces de préhension.

De préférence, la partie inférieure comporte exactement deux faces en vis-à-vis l'une de l'autre, configurées pour pouvoir être pincées entre le pouce et l'index.

Comme cela a été indiqué précédemment, lorsqu'une coupe se termine par une tige, tirer sur celle-ci pour retirer la coupe est inefficace et peut même être douloureux.

Grâce à l'invention, le retrait de la coupe peut être réalisé sans avoir à réaliser une telle traction sur la tige.

Au cas où une coupe conforme à l'invention comporte une tige, celle-ci doit de préférence servir uniquement à guider les doigts vers la partie inférieure de la coupe, afin que ceux-ci puissent se placer facilement sur les surfaces de préhension de la coupe. Il est possible de localiser facilement avec les doigts les faces de préhension de la coupe du fait de leur forme non convexe.

Pour permettre le retrait de la coupe hors du vagin, il s'est avéré qu'avantageusement une pression exercée sur la partie inférieure de la coupe permet de libérer ou désolidariser la coupe des parois du vagin, et ainsi de permettre son retrait.

En particulier, les surfaces de préhension peuvent être utilisées pour faire pour tourner la coupe autour de son axe et décoller ainsi celle-ci (ou du moins, désolidariser celle-ci) des parois du vagin.

Cette possibilité est permise par les surfaces de préhension, qui offrent une prise pour les doigts et permettent la transmission d'un couple de rotation (alors qu'inversement il est très difficile de faire tourner une coupe dont la partie inférieure a notamment une forme de révolution).

Le retrait de la coupe se fait donc en pressant la partie inférieure de la coupe, et éventuellement en faisant tourner celle-ci ; cette opération ou ces opérations permettent de décoller la coupe de la paroi vaginale ; on peut alors retirer facilement la coupe en pinçant les surfaces de préhension entre les doigts et en tirant la coupe hors du vagin.

Le fait que les surfaces de préhension sont non-convexes, comme défini précédemment, signifie qu'au moins dans un plan de section, la section de ces surfaces forme une courbe non convexe : par exemple un segment de droite, ou une courbe concave (dont le 'ventre' est dirigé vers l'intérieur de la coupe).

Les surfaces de préhension peuvent donc être notamment des surfaces sensiblement planes ; des surfaces en forme de tuile (avec le creux dirigé vers l'intérieur de la coupe) ; des surfaces en forme de creux (le creux pouvant notamment avoir une courbure complémentaire à la forme des doigts).

Dans un mode de réalisation, la partie inférieure évasée de la coupe est définie en partie (et éventuellement en majeure partie) par une surface de révolution, et les surfaces de préhension sont formées en creux relatif par rapport à ladite surface de révolution.

Dans un mode de réalisation, chacune des surfaces de préhension est une surface profilée. Par 'surface profilée', on désigne ici une surface engendrée par le déplacement d'une courbe génératrice le long d'une droite, ou selon une direction de déplacement donnée.

La direction de déplacement peut notamment être une direction perpendiculaire à l'axe de la coupe.

Par exemple, dans un mode de réalisation, la partie inférieure de la coupe est définie en partie par une surface de révolution, et les surfaces de préhension sont profilées suivant une direction de déplacement sensiblement circonférentielle, dans un plan de section horizontal, par rapport à cette surface de révolution.

Dans un mode de réalisation, la forme profilée présente une section sensiblement rectiligne. La courbe génératrice est alors sensiblement un segment de droite.

Dans un mode de réalisation, dans un plan perpendiculaire à un axe de la coupe à mi-hauteur des surfaces de préhension, la somme des angles au sommet des surfaces de préhension est supérieure à 90°. Les surfaces de préhension présentent alors une surface importante, ce qui facilite la tenue de la coupe et permet notamment de faire tourner celle-ci avec les doigts plus facilement que si les surfaces de préhension étaient plus petites.

Dans un mode de réalisation, lesdites surfaces de préhension sont agencées en dessous de 70%, et de préférence en dessous de 50%, de la hauteur d'une cavité intérieure de la coupe à partir d'un fond de ladite cavité. Ainsi, la présence des surfaces de préhension ne réduit pas sensiblement la capacité de la cavité intérieure de la coupe.

Dans un mode de réalisation, au moins une partie desdites surfaces de préhension présente un motif et/ou un grain.

Un motif désigne ici un relief particulier de la face ou des faces pouvant être identifié visuellement.

Un grain désigne ici un léger relief susceptible d'être perçu au toucher mais ne modifiant pas la forme générale de la surface ou des surfaces de préhension.

Le motif et/ou le grain permet de distinguer les surfaces de préhension du reste de la partie inférieure de la coupe (de la portion de la partie inférieure de la coupe qui est complémentaire aux surfaces de préhension) ; ce reste pouvant notamment être lisse ou sensiblement lisse, du moins par comparaison avec la partie des surfaces de préhension présentant le motif ou le grain.

Par ailleurs, un autre inconvénient évoqué par les utilisatrices ayant testé une coupe menstruelle est le confort d'utilisation, et notamment la présence d'une tige proéminente à la base de la coupe.

Pour remédier à ce problème, avantageusement, dans un mode de réalisation de l'invention la partie inférieure de la coupe est dépourvue de tige. L'absence de tige se traduit notamment par le fait que l'épaisseur de la coupe au point bas est inférieure à un tiers du diamètre maximal d'une cavité interne de la coupe. De préférence, l'épaisseur de la coupe au point bas est même inférieure à un cinquième du diamètre maximal de la cavité interne de la coupe.

De manière connue en soi, le haut d'une coupe menstruelle est généralement constitué par un anneau d'étanchéité.

Dans un mode de réalisation, la coupe menstruelle comporte en outre au moins un passage d'équilibrage de pression, aménagé entre l'intérieur et l'extérieur de la coupe, ledit au moins un passage débouchant à l'extérieur en dessous de l'anneau.

Aussi, lorsque l'on appuie sur les surfaces de préhension de la coupe pour retirer celle-ci, avantageusement on décolle la coupe de la paroi vaginale au niveau de l'orifice externe d'au moins un desdits passage. Une communication s'établit alors par ce passage entre l'intérieur et l'extérieur de la coupe : cette communication provoque immédiatement un équilibrage des pressions entre l'intérieur et l'extérieur de la coupe, ce qui facilite le retrait de la coupe en supprimant toute sensation de succion.

De préférence, le ou les orifices externes du ou des passages d'équilibrage de pression sont disposé(s) sensiblement au droit du ou des surfaces de préhension. En effet, lorsque l'on appuie sur une surface de préhension, la partie de la coupe qui est le plus décollée de la paroi vaginale est la partie de la coupe qui est située sensiblement au droit de la surface de préhension (ou des surfaces de préhension) sur laquelle (lesquelles) on a appuyé.

C'est donc dans cette portion de la surface externe de la coupe qu'il est le plus efficace de prévoir les orifices externes des passages d'équilibrage de pression de la coupe.

De préférence, le ou les orifices externes du ou des passages d'équilibrage de pression sont situés juste en dessous de l'anneau d'étanchéité, par exemple à une distance en dessous du bord inférieur de l'anneau inférieur à la moitié du rayon extérieur de l'anneau.

Dans un mode de réalisation, l'anneau d'étanchéité présente une surface extérieure cylindrique sur une hauteur supérieure à 1/10^{ème}, et de préférence à 1/5^{ème}, d'un rayon extérieur de l'anneau.

Ainsi, cette surface extérieure cylindrique forme une grande surface prévue pour être en contact avec la paroi du vagin, par contraste par exemple avec une surface extérieure en forme de tore, qui ne permettrait qu'un contact suivant une ligne avec la paroi du vagin. Par suite, un tel anneau confère à la coupe une particulièrement grande étanchéité.

Dans un mode de réalisation, l'anneau d'étanchéité présente une surface supérieure inclinée vers l'intérieur de la coupe, c'est-à-dire en forme d'entonnoir. Ce mode de réalisation facilite l'entrée du flux menstruel dans la coupe et réduit le risque qu'une partie du flux ne soit pas collectée par celle-ci.

### BREVE DESCRIPTION DES DESSINS

L'invention sera bien comprise et ses avantages apparaîtront mieux à la lecture de la description détaillée qui suit, de modes de réalisation représentés à titre d'exemples non limitatifs. La description se réfère aux dessins annexés, sur lesquels :
- les figures 1 et 2 sont deux vues schématiques de face et de côté respectivement d'une coupe menstruelle selon un premier mode de réalisation de l'invention ;
- les figures 3 et 4 sont des coupes de la coupe menstruelle des figures 1 et 2, respectivement dans un plan méridien et dans un plan transverse ;
- la figure 5 est une vue schématique en perspective de la coupe menstruelle des figures 1 et 2 ;
- les figures 6 et 7 sont des vues schématiques, respectivement en perspective et en coupe transverse, d'une coupe menstruelle dans un second mode de réalisation de l'invention ; et
- La figure 8 est une vue en coupe illustrant le retrait d'une coupe menstruelle.

### DESCRIPTION DETAILLEE DE L'INVENTION

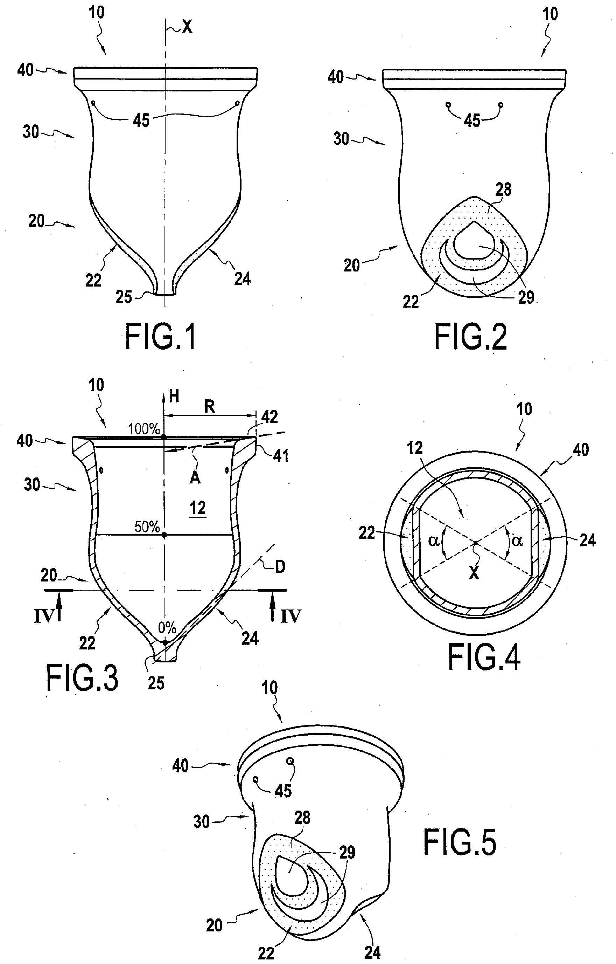

En faisant référence aux figures 1 à 7, deux coupes menstruelles 10 illustrant deux modes de réalisation de l'invention vont maintenant être décrites.

Ces deux coupes sont identiques à l'exception de certaines caractéristiques qui seront indiquées plus loin. Aussi, les éléments identiques ou similaires de ces deux coupes portent les mêmes références numériques.

Pour les éléments communs aux deux formes de coupe, la description qui suit est donc commune aux deux formes de coupe.

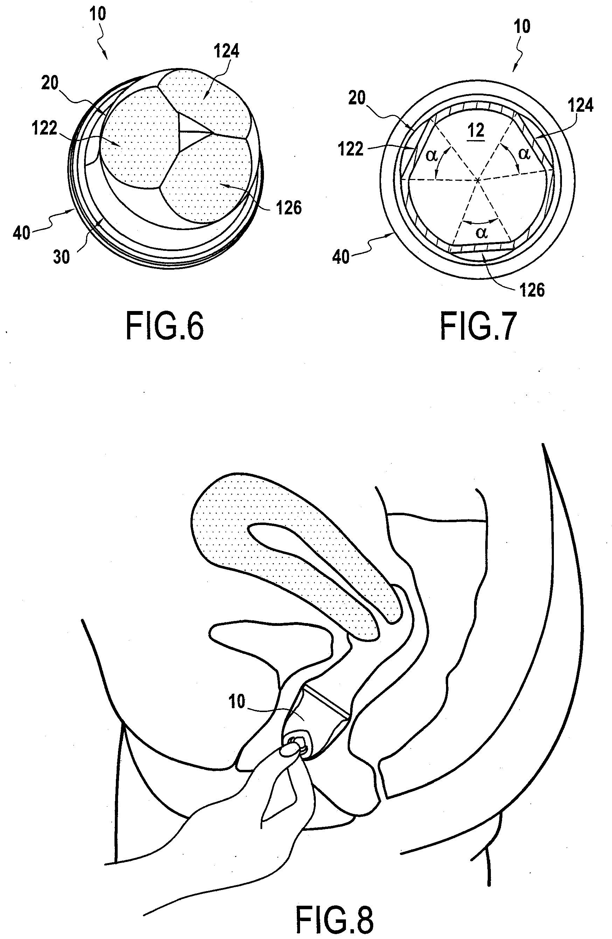

Ces deux formes sont illustrées respectivement par les figures 1 à 5 (premier mode de réalisation) et par les figures 6 et 7 (second mode de réalisation).

La coupe menstruelle 10 présente une partie inférieure évasée 20, une partie cylindrique 30 et un anneau d'étanchéité 40.

Une cavité intérieure 12 est formée à l'intérieur de la coupe 10.

La coupe 10 présente une forme globalement de révolution autour d'un axe X. On considère pour simplifier la description que cet axe X est vertical et que l'ouverture de la cavité 12 est disposée vers le haut.

La partie inférieure évasée 20 est la partie de la coupe dans laquelle le diamètre de la cavité intérieure 12 de la coupe diminue.

Dans le premier mode de réalisation (Fig.1 à 5), la partie inférieure évasée 20 comporte deux surfaces de préhension profilées 22 et 24, axisymétriques et disposées en vis-à-vis.

Dans le second mode de réalisation (Fig.6 et 7), la partie inférieure évasée 20 comporte trois surfaces de préhension profilées 122, 124 et 126, axisymétriques et disposées en vis-à-vis.

Les surfaces de préhension (22,24 ; 122,124,126) sont des surfaces profilées.

Dans les modes de réalisation présentés, les surfaces de préhension sont des surfaces profilées à section sensiblement rectiligne.

Ainsi dans le premier mode de réalisation, les surfaces de préhension 22,24 sont engendrées par le déplacement d'une courbe qui forme sensiblement un segment de droite suivant une direction perpendiculaire au plan de coupe de la figure 3. Sur la figure 3, on a représenté la droite D qui peut être considérée comme la droite support de ce segment de droite pour la surface 24.

Dans les modes de réalisation présentés, les surfaces de préhension de la coupe 10 sont agencées seulement sur la partie inférieure 20. Elles pourraient éventuellement s'étendre sur la partie cylindrique 30.

Ces surfaces de préhension s'étendent notamment seulement jusqu'à une hauteur inférieure à 50% de la hauteur H de la cavité intérieure 12 de la coupe. Cette hauteur H est mesurée à partir du fond de la coupe, et vaut 0% au niveau du fond de cette cavité, et 100% au niveau de l'ouverture supérieure de la cavité (Fig.3).

Les surfaces de préhension occupent une surface relativement importante sur la partie inférieure 20, pour permettre le placement des doigts et la préhension de la coupe.

Ainsi comme on le voit sur les figures 4 ou 7, dans une section de la coupe suivant un plan transverse à mi-hauteur des surfaces de préhension, la somme des angles au sommet α des surfaces de préhension est supérieure à 90°.

Les surfaces de préhension présentent un aspect visuel et tactile différent du reste de la surface externe de la partie inférieure 20.

Ainsi dans le premier mode de réalisation, les surfaces de préhension 22 et 24 présentent un grain sur des surfaces grainées 28. Ce grain est obtenu de manière classique par des surfaces grainées de forme complémentaire, prévues dans les moules de fabrication de la coupe 10.

Les surfaces de préhension 22 et 24 sont entièrement grainées dans les surfaces 28, à l'exception de réservations 29. Ces réservations 29 sont les portions de la surface de préhension 22 et 24 qui ne sont pas grainées contrairement au reste des surfaces 22 et 24, qui constitue les surfaces grainées 28. Les réservations 29 restent donc lisses comme le reste de la surface externe de la coupe 10.

L'alternance de surfaces grainées et de surfaces lisses (surfaces 28/29) forme un motif qui permet la reconnaissance tactile et/ou visuelle des surfaces de préhension 22 et 24.

Dans le second mode de réalisation, les surfaces de préhension 122,124,126 sont entièrement grainées, et ne comportent pas de réservations lisses.

Avantageusement, la présence des surfaces de préhension dans la partie inférieure de la coupe 10 rend la tige totalement inutile.

Dans le premier mode de réalisation, le bas de la coupe 10 comporte juste une petite pointe 25, en forme de crête. Celle-ci permet de localiser l'extrémité de la coupe et facilite le placement des doigts sur les surfaces de préhension.

La partie haute de la coupe 10, au voisinage de l'anneau d'étanchéité 40, comporte des passages d'équilibrage de pression 45. Ces passages présentent des orifices externes (également notés 45) qui sont situés globalement au droit des surfaces de préhension 22, 24 (comme on peut le voir notamment sur la figure 2). Ces passages sont des passages qui traversent la paroi de la coupe et relient donc la cavité interne 12 de la coupe à l'extérieur de celle-ci.

L'anneau d'étanchéité présente une surface externe cylindrique 41. Cette surface s'étend verticalement (suivant l'axe X) suivant une certaine hauteur. Cette hauteur est supérieure à 1/10^{ème} du rayon externe R de l'anneau 40 (Fig.3).

La surface externe cylindrique 41 a pour objectif d'améliorer l'étanchéité en augmentant la surface de contact entre le bord supérieur et extérieur de la coupe et la paroi du vagin ; et maintenir une rigidité suffisante, à la fois lors du pliage et du déploiement de la coupe une fois installée.

L'anneau 40 présente de plus une surface supérieure 42 inclinée vers l'intérieur de la coupe. La forme de cette surface 42 est matérialisée par la flèche A sur la figure 3. La surface supérieure 42 présente donc une pente descendante vers l'intérieur de la coupe, depuis l'arête formée à l'extrémité supérieure de la surface cylindrique extérieure 41, jusqu'à la jonction de l'anneau 40 avec la cavité interne 12 de la coupe.

Grâce à l'inclinaison vers l'intérieur (radialement) de cette surface, un flux menstruel qui se trouverait sur cette surface supérieure de l'anneau 40 s'écoulerait à l'intérieur, et non à l'extérieur, de la cavité 12 de la coupe.

La figure 8 présente de manière schématique une opération de retrait de la coupe 10.

Pour retirer celle-ci, il faut pincer la partie inférieure de la coupe entre les doigts. On peut faire tourner la coupe 10 autour de son axe pour la désolidariser du vagin ; on tire alors la coupe vers le bas et on l'extrait ainsi à l'extérieur du vagin.

Quoique la présente invention ait été décrite en se référant à des exemples de réalisation spécifiques, il est évident que des différentes modifications et changements peuvent être effectués sur ces exemples sans sortir de la portée générale de l'invention telle que définie par les revendications. En outre, des caractéristiques individuelles des différents modes de réalisation évoqués peuvent être combinées dans des modes de réalisation additionnels. Par conséquent, la description et les dessins doivent être considérés dans un sens illustratif plutôt que restrictif.

## Revendications

1. Coupe menstruelle (10) présentant une partie inférieure évasée (20), **caractérisée en ce qu'**elle comporte une pluralité de surfaces de préhension (22,24 ; 122,124,126) non convexes, réparties de manière sensiblement axisymétrique au moins sur ladite partie inférieure évasée (20), et configurées pour pouvoir être pincées entre des doigts afin de faciliter le retrait de la coupe.

2. Coupe menstruelle (10) selon la revendication 1, dans laquelle chacune desdites surfaces de préhension (22,24 ; 122,124,126) est une surface profilée.

3. Coupe menstruelle (10) selon revendication 1 ou 2, dans laquelle, dans un plan perpendiculaire à un axe de la coupe à mi-hauteur des surfaces de préhension, la somme des angles au sommet (α) des surfaces de préhension est supérieure à 90°.

4. Coupe menstruelle (10) selon l'une quelconque des revendications 1 à 3, dans laquelle lesdites surfaces de préhension sont agencées en dessous de 70%, et de préférence en dessous de 50%, de la hauteur (H) d'une cavité intérieure (12) de la coupe à partir d'un fond de ladite cavité.

5. Coupe menstruelle (10) selon l'une quelconque des revendications 1 à 4, dont au moins une partie (28) desdites surfaces de préhension présente un motif et/ou un grain.

6. Coupe menstruelle (10) selon l'une quelconque des revendications 1 à 5, dont la partie inférieure (20) est dépourvue de tige.

7. Coupe menstruelle (10) selon l'une quelconque des revendications 1 à 6, comprenant en outre un anneau d'étanchéité (40) situé en haut de la coupe, et au moins un passage d'équilibrage de pression (45) aménagé entre un extérieur et un intérieur de la coupe, ledit au moins un passage (45) débouchant à l'extérieur en dessous de l'anneau (40).

8. Coupe menstruelle (10) selon l'une quelconque des revendications 1 à 7, comprenant en outre un anneau d'étanchéité (40) situé en haut de la coupe, ledit anneau présentant une surface extérieure cylindrique (41) sur une hauteur supérieure à 1/10^{ème}, et de préférence à 1/5^{ème}, d'un rayon extérieur (R) de l'anneau.

9. Coupe menstruelle (10) selon l'une quelconque des revendications 1 à 8, comprenant en outre un anneau d'étanchéité (40) situé en haut de la coupe, ledit anneau présentant une surface supérieure (42) inclinée vers l'intérieur de la coupe.

10. Coupe menstruelle (10) selon l'une quelconque des revendications 1 à 9, comportant exactement soit deux surfaces de préhension (22,24), soit trois surfaces de préhension (122,124,126).

## Patentansprüche

1. Menstruationstasse (10) mit einem aufgeweiteten Unterteil (20), **dadurch gekennzeichnet, dass** sie eine Vielzahl von nicht konvexen Griffflächen (22, 24; 122, 124, 126) umfasst, die zumindest auf dem aufgeweiteten Unterteil (20) im Wesentlichen achsensymmetrisch verteilt und zwischen den Fingern einklemmbar sind, um das Entfernen der Tasse zu erleichtern.

2. Menstruationstasse (10) nach Anspruch 1, wobei jede der Griffflächen (22, 24; 122, 124, 126) eine profilierte Oberfläche ist.

3. Menstruationstasse (10) nach Anspruch 1 oder 2, wobei in einer Ebene senkrecht zu einer Achse des Abschnitts in mittlerer Höhe der Griffflächen die Summe der Winkel an der Oberseite (a) der Griffflächen größer als 90° ist.

4. Menstruationstasse (10) nach einem der Ansprüche 1 bis 3, wobei die Griffflächen unterhalb von 70% und vorzugsweise unterhalb von 50% der Höhe (H) eines inneren Hohlraums (12) der Tasse von einem Boden des Hohlraums angeordnet sind,

5. Menstruationstasse (10) nach einem der Ansprüche 1 bis 4, wobei mindestens ein Teil (28) der Griffflächen ein Muster und/oder eine Maserung aufweist.

6. Menstruationstasse (10) nach einem der Ansprüche 1 bis 5, wobei das Unterteil (20) keinen Schaft aufweist.

7. Menstruationstasse (10) nach einem der Ansprüche 1 bis 6, ferner umfassend einen Dichtungsring (40), der sich an der Oberseite der Tasse befindet, und mindestens einen Druckausgleichskanal (45), der zwischen einer Außenseite und einer Innenseite der Tasse angeordnet ist, wobei der mindestens eine Kanal (45) unter dem Ring (40) herausführt.

8. Menstruationstasse (10) nach einem der Ansprüche 1 bis 7, ferner umfassend einen Dichtungsring (40), der sich an der Oberseite der Tasse befindet, wobei der Ring eine zylindrische Außenfläche (41) über eine Höhe von mehr als einem Zehntel, vorzugsweise einem Fünftel eines Außenradius (R) des Rings aufweist.

9. Menstruationstasse (10) nach einem der Ansprüche 1 bis 8, ferner umfassend einen Dichtungsring (40), der sich an der Oberseite der Tasse befindet, wobei der Ring eine obere Oberfläche (42) aufweist, die zur Innenseite der Tasse geneigt ist.

10. Menstruationstasse (10) nach einem der Ansprüche 1 bis 9, der genau entweder zwei Griffflächen (22, 24) oder drei Griffflächen (122, 124, 126) umfasst.

## Claims

1. A menstrual cup (10) having a flared bottom portion (20), **characterized in that** it includes a plurality of non-convex grip surfaces (22, 24; 122, 124, 126) that are distributed in substantially axisymmetric manner at least on said flared bottom portion (20) and that are configured so as to be able to be pinched between fingers in order to facilitate removal of the cup.

2. A menstrual cup (10) according to claim 1, wherein each of said grip surfaces (22, 24; 122, 124, 126) is a profiled surface.

3. A menstrual cup (10) according to claim 1 or claim 2, wherein, in a plane perpendicular to an axis of the cup at mid-height of the grip surfaces, the sum of the angles at the vertex (a) of the grip surfaces is greater than 90°.

4. A menstrual cup (10) according to any one of claims 1 to 3, wherein said grip surfaces are arranged at below 70%, and preferably below 50%, of the height (H) of an inside cavity (12) of the cup from a bottom of said cavity.

5. A menstrual cup (10) according to any one of claims 1 to 4, wherein at least a portion (28) of said grip surfaces presents a pattern and/or a grain.

6. A menstrual cup (10) according to any one of claims 1 to 5, wherein the bottom portion (20) does not have a stem.

7. A menstrual cup (10) according to any one of claims 1 to 6, further including a sealing ring (40) situated at a top of the cup, and at least one pressure-equalizing passage (45) made between an outside and an inside of the cup, said at least one passage (45) opening out below the ring (40).

8. A menstrual cup (10) according to any one of claims 1 to 7, further including a sealing ring (40) situated at a top of the cup, said ring having an outer cylindrical surface (41) over a height that is greater than 1/10^{th}, and preferably greater than 1/5^{th}, of an outside radius (R) of the ring.

9. A menstrual cup (10) according to any one of claims 1 to 8, further including a sealing ring (40) situated at a top of the cup, said ring having a top surface (42) that slopes towards the inside of the cup.

10. A menstrual cup (10) according to any one of claims 1 to 9, including exactly either two grip surfaces (22, 24), or three grip surfaces (122, 124, 126).
